# NEUE EUROPÄISCHE PATENTSCHRIFT

(11) **EP 0 599 214 B2**
(45) Veröffentlichungstag und Bekanntmachung des Hinweises auf die Entscheidung über den Einspruch: **24.01.2001**
(45) Hinweis auf die Patenterteilung: 13.08.1997
(21) Anmeldenummer: 93118646.4
(22) Anmeldetag: 19.11.1993
(51) Int. Cl.: C07C 45/43, C07C 47/54, C07C 47/55

(54) **Verfahren zur kontinuierlichen Herstellung von aromatischen Aldehyden**
Process for the continuous preparation of aromatic aldehydes
Procédé de préparation en continu d'aldéhydes aromatiques

(30) Priorität: 26.11.1992 DE 4239736
(43) Veröffentlichungstag der Anmeldung: 01.06.1994
(73) Patentinhaber: Clariant GmbH, 65929 Frankfurt am Main (DE)
(72) Erfinder: Billeb, Gilbert, Dr., D-65779 Kelkheim/Ts (DE); Folz, Georg, Dr., D-60529 Frankfurt/Main (DE)

(56) Entgegenhaltungen:
- DE-A- 2 026 817
- DE-A- 2 044 832
- NL-A- 7 215 589
- US-A- 3 524 885
- DATABASE WPI Week 8604, Derwent Publications Ltd., London, GB; AN 86-025603 & JP-A-60 248 640 (HODOGAYA CHEM IND KK) 9. Dezember 1985
- DATABASE WPI Week 8550, Derwent Publications Ltd., London, GB; AN 85-313611 & JP-A-60 218 349 (HODOGAYA CHEM IND KK) 1. November 1985
- CHEM. ABSTR., Bd. 84, 1976, Zusammenfassung Nr. 135232g & Pr. Inst. Przem. Org., 1974 (Pub. 1975) (6), 1-9
- ULLMANN, Enzyklopädie der technischen Chemie, 4. Aufl., Bd. A3, S. 465, 466, 1985
- CHEM. ABSTR., Bd. 104, 1986, Zusammenfassung Nr. 168138r & JP-A-60 248640
- CHEM. ABSTR., Bd. 87, 1977, Zusammenfassung Nr. 84685j & JP-A-77 025733
- CHEM. ABSTR., Bd. 86, 1977, Zusammenfassung Nr. 43407f & JP-A-76 06129
- WINNACKER-KÜCHLER, CHEMISCHE TECHNOLOGIE, 4. AUFL., BD. 6, S. 203

## Beschreibung

Gegenstand der vorliegenden Erfindung ist ein verbessertes Verfahren zur kontinuierlichen Herstellung von halogenierten aromatischen Aldehyden in sehr guten Ausbeuten durch Hydrolyse der entsprechenden dichlormethylsubstituierten Aromaten mit wäßrigen Lösungen bestimmter Katalysatoren.

Aromatische Aldehyde sind wichtige Ausgangsprodukte zur Herstellung zahlreicher Verbindungen auf dem Gebiet der Pflanzenschutzmittel und Pharmazeutika.

Aromatische Aldehyde werden im allgemeinen durch Verseifung der entsprechenden dichlormethylsubstituierten Aromaten mit Wasser in Gegenwart von Säuren oder Metallsalzen gemäß der Reaktionsgleichung 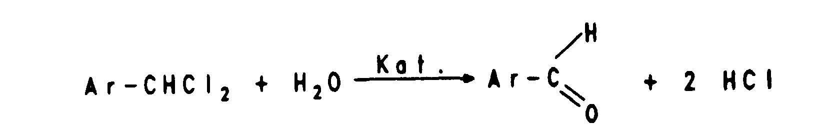 hergestellt (vgl. Houben-Weyl, VII,S. 211 ff.), wobei die Reaktion meist diskontinuierlich durchgeführt wird. Diese Fahrweise weist jedoch die nachstehenden Nachteile auf:
1. Die Reaktionszeiten sind in der Regel recht lang, was große Apparatedimensionen erforderlich macht.
2. Speziell größere Produktionsansätze sind mit einem erhöhten Sicherheitsrisiko behaftet, da mitunter bei verzögertem Einsetzen der Verseifung die Reaktion außer Kontrolle geraten kann.
3. Problematisch ist der Einsatz von Katalysatoren, wie beispielsweise Eisensalzen, die unerwünschte Nebenreaktionen (Friedel-Crafts-Reaktionen, Polymerisationen) katalysieren können, weil bei der Batch-Fahrweise zu Beginn der Reaktion hohe Konzentrationen an dichlormethylsubstituiertem Aromat vorliegen, die derartige Reaktionen begünstigen. Diese Reaktionen können oft unkontrolliert ablaufen und führen letztlich zu Ausbeuteminderung oder Verharzung des Ansatzes.

Es bestand daher in mehrfacher Hinsicht ein Bedürfnis nach einem verbesserten Verfahren zur Herstellung von gegebenenfalls substituierten aromatischen Aldehyden, bevorzugt Benzaldehyden, welches die genannten Nachteile nicht besitzt.

In der Literatur sind bereits mehrere Verfahren zur Herstellung von Benzaldehyden nach dem vorstehend angegebenen Reaktionsschema beschrieben:

In der japanischen Bekanntmachung 17217/62, Hodogaya Chemical Co., Ltd., wird die kontinuierliche Verseifung verschiedener Benzalchloride in Gegenwart großer Mengen Schwefelsäure dargestellt. Dieses Verfahren ist aus ökologischer und ökonomischer Sicht nach wie vor bedenklich, da enorme Mengen Schwefelsäure benötigt werden, die letztlich ins Abwasser gelangen oder aufwendig regeneriert werden müssen. Der Umsatz bei diesem Verfahren beträgt maximal 97 %, was einen großen Destillationsaufwand zur Abtrennung von überschüssigem Benzalchlorid nötig macht. Darüber hinaus ist es sehr schwierig, auf diesem Wege ein chlorfreies Produkt zu erhalten. Die Ausbeute an Aldehyd, bezogen auf eingesetztes Benzalchlorid, ist mit 87,5 % d.Th. sehr mäßig.

In GB 2 103 208 wird eine Methode zur Herstellung von Benzaldehyden in der Gasphase beschrieben. Diese Methode eignet sich nur für thermisch stabile Aldehyde und ist energetisch sowie im Hinblick auf die Wirkstoffe (230°C, HCI-Abgas) nicht unproblematisch.

In EP 64486 wird ein kontinuierliches Verfahren zur Herstellung von Benzaldehyden beschrieben, welches dadurch gekennzeichnet ist, daß in einer mehrstufigen Gegenstrom-Extraktionsanlage die Verseifung in einem inerten Lösungsmittel mit einer wäßrigen Lösung einer Base durchgeführt wird. Dieses Verfahren ist aufgrund der Entstehung von stark kochsalz-haltigen Abwässern sowie der Notwendigkeit großer Lösemittelkreisläufe ökologisch problematisch und aufwendig.

In JP-A-60 248 640 und JP-A-60 218-349 ist der Einsatz fester Zinksalze, in NL-A-7 215 589 die Hydrolyse mit wäßrigen Zinksalzen beschrieben, jedoch nur unter der Bedingung, daß vor der Reaktion mindestens ein Teil des Katalysators in fester Form zugesetzt wird Der Einsatz von festen Salzen ist jedoch verfahrenstechnisch von großem Nachteil.

Es wurde nun gefunden, daß man aromatische Aldehyde der allgemeinen Formel (1) 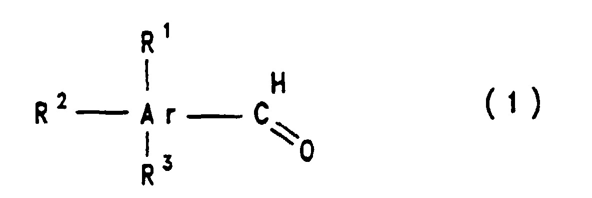 in welcher Ar einen Arylrest von 6 bis 14 Kohlenstoffatomen, beispielsweise und vorzugsweise den Benzolrest darstellt, R¹, R², R³ unabhängig voneinander Wasserstoff-, Fluor-, Chlor- oder Bromatome, vorzugsweise Wasserstoff-, Fluor- oder Chloratome, bedeuten, vorteilhaft kontinuierlich herstellen kann, indem man dichlormethylsubstituierte Aromaten der allgemeinen Formel (2) 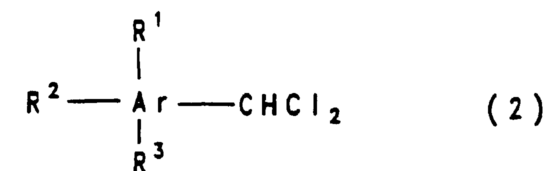 in welcher Ar, R¹, R², R³ die vorstehend genannten Bedeutungen haben, durch Zudosieren des gesamten Katalysators in Form einer wäßrigen Lösung eines oder mehrerer Katalysatoren der allgemeinen Formel (3)

MXₙ (3)

in welcher M ein Übergansmetall aus der Reihe Nickel, Kupfer und Zink, vorzugsweise aus der Reihe Kupfer und Zink, besonders bevorzugt Zink, bedeutet, X für F, Cl, Br, I, OH, SO₄, PO₄ oder NO₃, vorzugsweise Cl, Br, OH, SO₄, PO₄ oder NO₃ steht und n in Abhängigkeit von der Oxidationsstufe des Übergangsmetalls M die Zahl 1, 2, 3 oder 4 bedeutet, einer Konzentration von 1 bis 50 Gew.%, vorzugsweise 1, 2 bis 15 Gew.%, bezogen auf das Gewicht des Wassers der Katalysatorlösung, bei Temperaturen von 70° bis 160°C, vorzugsweise, 100° bis 140°C in Gegenwart von zur Hydrolyse erforderlichem Wasser in stöchiometrischer Menge bis zu einem Überschuß von etwa 100 %, bezogen auf die Verbindung der Formel (2), hydrolysiert.

Der als Ausgangsverbindung dienende dichlormethylsubstituierte Aromat der genannten allgemeinen Formel (2) kann in reiner Form oder im Gemisch mit dem entsprechenden trichlormethylsubstituierten Aromat eingesetzt werden, was die gleichzeitige Herstellung von aromatischem Aldehyd und aromatischer Carbonsäure ermöglicht. Setzt man ein Gemisch ein, so kann dieses bis zu 50 Gew.%, beispielsweise 0,5 bis 30 Gew.%, des entsprechenden trichlormethylsubstituierten Aromaten enthalten. Geringe Verunreinigungen der Ausgangsverbindung der Formel (2) mit dem entsprechenden monochlormethylsubstituierten Aromat stören bei der Verseifungsreaktion nicht.

Wird ein Gemisch aus einem dichlormethylsubstituierten Aromat, beispielsweise Benzalchlorid, und einem trichlormethylsubstituierten Aromat, beispielsweise Benzotrichlorid, eingesetzt, so kann nach Beendigung der Reaktion vor oder nach einer Destillation die gebildete aromatische Carbonsäure durch alkalische Wäsche herausgelöst und aus der resultierenden Lösung durch Ansäuern ausgefällt und isoliert werden.

Beim erfindungsgemäßen Verfahren ist die Ausbeute an reinem Aldehyd, beispielsweise an gegebenenfalls halogeniertem Benzaldehyd, > 95 %, bezogen auf eingesetztes, gegebenenfalls halogeniertes Benzalchlorid. Im Falle der Anwendung eines Gemisches von beispielsweise Benzalchlorid und Benzotrichlorid ist die Ausbeute an Carbonsäure > 95 % und die Ausbeute an Aldehyd > 95 % nach Abtrennung und Reinigung der Produkte.

Das erfindungsgemäße Verfahren zeichnet sich insbesondere dadurch aus, daß es mit geringen Mengen an Metallsalzen, bezogen auf eingesetztes Ausgangsprodukt der Formel (2), auskommt (0,1 bis 1,5 g/100g). Im Vergleich hierzu benötigt man beim Verfahren von JP 17217/62 etwa 40 bis 50 g konz. Schwefelsäure/100 g Ausgangsverbindung. Obendrein ist die Verwendung von wäßrigen Zinksalzlösungen gefahrlos möglich. Zinksalz-Lösungen sind literaturangaben zu folge im Gegensatz zu festen Zinksalzen im Batch-Verfahren nicht katalytisch wirksam (J. Legocki et al., Pr. Inst. Przem. Org. 1974, 6, 1-9). Umso überraschender ist es, daß bei der kontinuierlichen Fahrweise die Verseifung mit beispielsweise 10 prozentiger wäßriger Zinkchlorid-Lösung glatt und in sehr hohen Umsätzen und Ausbeuten verläuft, die Verwendung von festem Zinksalz als Katalysator wäre bei kontinuierlicher Fahrweise aufgrund von Dosierungsproblemen nur sehr schwer realisierbar.

Eine bevorzugte Ausführungsform des erfindungsgemäßen Verfahrens besteht somit auch darin, daß man die Verseifung der Verbindungen der genannten Formel (2) mit einer wäßrigen Lösung von Zinksalzen mit den vorstehend genannten Anionen als Katalysator durchgeführt.

Es ist zweckmäßig, das erfindungsgemäße Verfahren, d.h. die Hydrolyse der Verbindungen der genannten allgemeinen Formel (2), in einer Rührkesselkaskade, bestehend aus 2 bis 5, vorzugsweise 2 bis 3, hintereinander geschalteten, heizbaren Rührkesseln durchzuführen, wobei man in den ersten Kessel den dichlormethylsubstituierten Aromat der Formel (2), beispielsweise das Benzalchlorid, in reiner (oder mit geringen Mengen monochlormethylsubstituiertem Aromat, beispielsweise Benzylchlorid verunreinigter) Form oder gegebenenfalls ein Gemisch aus dichlormethylsubstituiertem Aromat, beispielsweise Benzalchlorid, und dem entsprechenden trichlormethylsubstituierten Aromat, beispielsweise Benzotrichlorid, die wäßrige Katalysatorlösung eindosiert und das resultierende Reaktionsgemisch durch Überlauf oder Pumpen in die nachgeschalteten Kessel gelangen läßt. In die nachfolgenden Kessel kann zusätzlich Wasser oder wäßrige Katalysatorlösung nach Bedarf eindosiert werden.

Das für die Hydrolyse erforderliche Wasser wird in stöchiometrischer Menge oder in einem Überschuß bis zu etwa 100 %, vorzugsweise bis zu etwa 15 %, bezogen auf die Ausgangsverbindung der genannten Formel (2), eingesetzt.

Die Reaktionstemperatur in allen Kesseln beträgt 70 bis 160°C, vorzugsweise 100 bis 140°C. Die mittlere Verweilzeit beträgt in jedem Kessel 30 bis 240 min., vorzugsweise 40 bis 80 min., wobei die Verweilzeit in den einzelnen Kesseln auch unterschiedlich sein kann.

Die Reaktion wird vorzugsweise so geführt, daß im ersten Kessel bereits ein Umsatz von > 85 %, vorzugsweise > 90 % erreicht wird.

Das Rohprodukt am Reaktorausgang der Kaskade weist einen Gehalt an dichlormethylsubstituiertem Aromat, beispielsweise Benzalchlorid, von weniger als 1 %, vorzugsweise weniger als 0,1 %, und einen Gehalt an aromatischem Aldehyd, beispielsweise Benzaldehyd, ohne Berücksichtigung von überschüssigem Wasser von > 99 % auf. Es kann nach Entwässerung destilliert oder roh weiterverarbeitet werden.

Die nachstehenden Beispiele dienen zur Erläuterung des Verfahrens, ohne es darauf zu beschränken. Vorbemerkung zu den Ausführungsbeispielen:

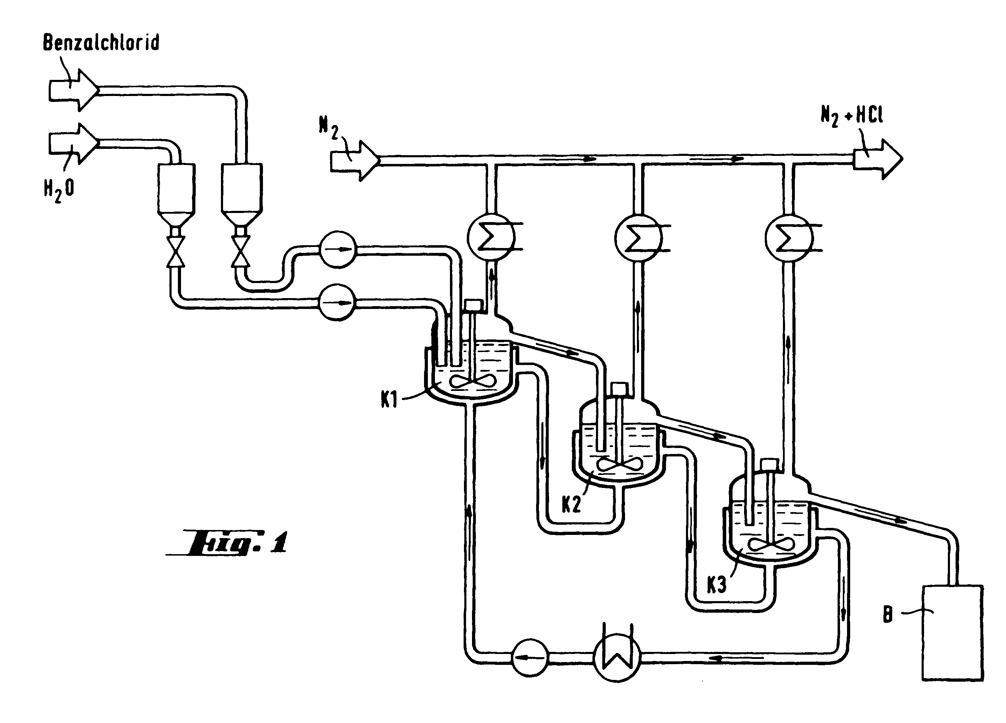

Alle nachstehenden Beispiele wurden in einer Rührkesselkaskade, bestehend aus drei hintereinandergeschafteten, heizbaren Rührkesseln K1 bis K3, entsprechend beigefügter Zeichnung (vgl. FIGUR 1) durchgeführt. Die Kaskade wurde jeweils vor dem eigentlichen Versuch mit aromatischem Aldehyd der genannten allgemeinen Formel (1) bzw. mit fertigem Reaktionsgemisch gefüllt und auf Reaktionstemperatur gebracht. (In FIGUR 1 stellt B die Produktvorlage dar.)

### Beispiel 1 (Verseifung von o-Chlorbenzalchlorid)

Die Kessel K1 bis K3 wurden mit diskontinuierlich hergestelltem Reaktionsgemisch mit einem Gehalt an o-Chlor-benzaldehyd von etwa 98 % bis zum Überlauf gefüllt und auf 130°C erhitzt. In den Kessel K1 wurden anschließend o-Chlorbenzalchlorid mit einer Dosierrate von 268 ml/h (369,8 g, 1,89 Mol) und 10 %ige Zinkchloridlösung mit einer Dosierrate von 36 ml/h eindosiert. Der aus der Kaskade austretende Volumenstrom betrug 220 ml/h und die mittlere Verweilzeit in jedem Kessel etwa 60 Minuten. Nach 5 h befand sich die Kaskade in einem Gleichgewicht. Der Umsatz im Kessel K1 betrug 99,3 %. Das im Gleichgewicht aus der Kaskade austretende Rohprodukt wies einen Gehalt an o-Chlorbenzalchlorid von < 0,04 % auf (Kapillar-GC-Flächenprozent) und war ohne Berücksichtigung von überschüssigem Wasser 99,5 %ig an o-Chlorbenzaldehyd. Die Ausbeute an reinem o-Chlorbenzaldehyd betrug etwa 256 g/h (1,82 Mol/h, 96 % d.Th.).

### Beispiel 2 (Verseifung von o-Chlorbenzalchlorid)

Im Gegensatz zu Beispiel 1 wurde die Verweilzeit durch Erhöhung der Stoffströme auf 47 min. pro Kessel verkürzt. Die Kaskade war zu Beginn mit fertigem Reaktionsgemisch aus Beispiel 1 gefüllt. In Kessel K1 wurde o-Chlorbenzalchlorid mit einer Dosierrate von 356 ml/h (491 g/h, 2,5 Mol/h) und 10 %ige Zinkchloridlösung mit einer Dosierrate von 43,6 ml/h eindosiert. Zusätzlich wurde in Kessel K2 10 %ige Zinkchloridlösung mit einer Dosierrate von 3,6 ml/h zudosiert. Nach 3 h war die Kaskade im Gleichgewicht (GC-Kontrolle). Der austretende Stoffstrom betrug 294 ml/h. Im Gleichgewicht betrug der Gehalt an o-Chlorbenzalchlorid im Kessel K1 ca. 6 %, im Kessel K2 ca. 0,7 % und im Kessel K3 bzw. im austretenden Strom ca. 0,05 % (alle Angaben Kapillar-GC-Flächenprozent). Der Gehalt an o-Chlorbenzaldehyd im austretenden Strom betrug 99,7 % (GC) ohne Berücksichtigung überschüssigen Wassers. Die Ausbeute an reinem o-Chlor-benzaldehyd betrug 280 ml/h (339 g/h, 2,41 Mol/h, 96 % d.Th.).

### Beispiel 3 (Verseifung von p-Chlorbenzalchlorid)

Die Verseifung von p-Chlorbenzalchlorid wurde entsprechend Beispiel 1 bei einer Reaktionstemperatur von 100°C bei gleichen Stoffströmen und Verweilzeiten durchgeführt. Im Gleichgewicht war der Umsatz an p-Chlorbenzalchlorid in Kessel K1 der Kaskade ca. 98 % und in Kessel K2 quantitativ. Das Rohprodukt wies einen Gehalt an p-Chlorbenzaldehyd von > 99 % auf. Die Ausbeute an reinem p-Chlorbenzaldehyd betrug 255 g/h (96 % d.Th.).

### Beispiel 4 (Verseifung eines Gemisches aus o-Chlorbenzotrichlorid und o-Chlorbenzalchlorid)

Die Verseifung wurde entsprechend Beispiel 1 mit einer Mischung aus 76,1 Gew.% o-Chlorbenzalchlorid und 23,5 Gew.% o-Chlorbenzotrichlorid bei gleicher Temperatur und gleichen Stoffströmen durchgeführt. Es wurden keine signifikanten Unterschiede in der Verseifungsgeschwindigkeit festgestellt. Das o-Chlorbenzotrichlorid verseift unter den Reaktionsbedingungen vollständig zur o-Chlorbenzoesäure. Durch kontinuierliches Waschen des Verseifungsgemisches mit 4 %iger Sodalösung wurde die o-Chlorbenzoesäure herausgelöst und der rohe o-Chlorbenzaldehyd in einem Phasenscheider abgetrennt und zur Reinigung bei 10 torr destilliert. Die sodaalkalische Lösung wurde mit 30 %iger Salzsäure angesäuert, wobei die o-Chlorbenzoesäure ausfiel und abfiltriert wurde. Die Ausbeute an o-Chlorbenzaldehyd betrug 95,1 % d.Th., bezogen auf eingesetztes o-Chlorbenzalchlorid, die der o-Chlorbenzoesäure 99 % d.Th., bezogen auf eingesetztes o-Chlorbenzotrichlorid.

### Beispiel 5

Die Verseifung von o-Chlorbenzalchlorid wurde analog Beispiel 1 mit 2%iger Zinkchlorid-Lösung durchgeführt. Es wurden keine signifikant unterschiedlichen Ergebnisse erhalten.

### Beispiel 6

Die Verseifung von o-Chlorbenzalchlorid wurde analog Beispiel 1 mit 10%iger Zn(OH)₂-Lösung durchgeführt. Es wurde kein Unterschied zur Verwendung von Zinkchlorid festgestellt.

### Beispiel 7

Analog Beispiel 1 wurde die Verseifung von 2-Chlor-6-fluorbenzalchlorid bei 140°C durchgeführt. Der Eingangsstrom an 2-Chlor-6-fluorbenzalchlorid betrug 268 mg/h (396,6 g/h, 1,86 Mol/h). Die 10%ige Zinkchlorid-Lösung wurde mit einer Dosierrate von 36 ml/h eindosiert. Der aus der Kaskade austretende Volumenstrom betrug 242 ml/h und die mittlere Verweilzeit in jedem Kessel ca. 66 min. Das austretende Rohprodukt hatte einen Gehalt an 2-Chlor-6-fluorbenzaldehyd von 99,4 %. Die Ausbeute an reinem 2-Chlor-6-fluorbenzaldehyd betrug 280 g/h (1,77 mol, 95 % d.Th.).

### Beispiel 8

Die Verseifung von 4-Fluorbenzalchlorid wurde analog Beispiel 1 bei 100°C durchgeführt. Der Eingangsvolumenstrom an 4-Fluorbenzalchlorid betrug 268 ml/h (359 g/h, 2,0 Mol/h). Der austretende Volumenstrom betrug 213 ml/h und war > 99%ig an 4-Fluorbenzaldehyd. Die Ausbeute an reinem 4-Fluorbenzaldehyd betrug 235 g/h (94,7 d.Th.).

## Patentansprüche

1. Verfahren zur kontinuierlichen Herstellung aromatischer Aldehyde der allgemeinen Formel (1) 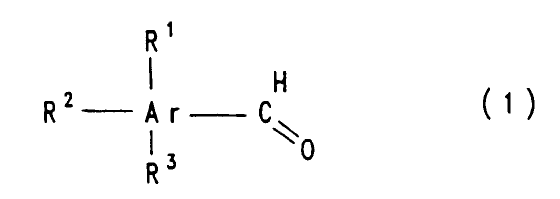 in welcher Ar einen Arylrest von 6 bis 14 Kohlenstoffatomen darstellt, R¹, R² und R³ unabhängig voneinander Wasserstoff-, Fluor-, Chlor- oder Bromatome bedeuten, dadurch gekennzeichnet, daß man dichlormethylsubstituierte Aromaten der allgemeinen Formel (2) 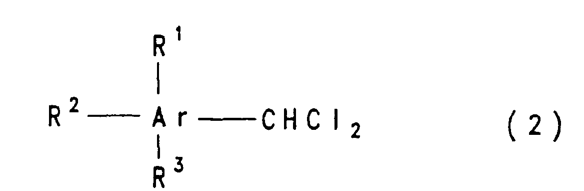 in welcher Ar, R¹, R², R³ die vorstehend genannten Bedeutungen haben durch Zudosieren des gesamten Katalysators in Form einer wäßrigen Lösung eines oder mehrerer Katalysatoren der allgemeinen Formel (3)
MXₙ (3)
in welcher M ein Übergangsmetall aus der Reihe Nickel, Kupfer und Zink bedeutet, X für F, Cl, Br, I, OH, SO₄, PO₄ oder NO₃ steht, und n in Abhängigkeit von der Oxidationsstufe des Übergangsmetalls M die Zahl 1, 2, 3 oder 4 bedeutet, einer Konzentration von 1 bis 50 %, bezogen auf das Gewicht des Wassers der Katalysatorlösung, bei Temperaturen von 70° bis 160°C kontinuierlich hydrolysiert.

2. Verfahren nach Anspruch 1, dadurch gekennzeichnet, daß in den allgemeinen Formeln (1) und (2) Ar den Rest des Benzols, Naphthalins, Anthracens oder Phenanthrens darstellt.

3. Verfahren nach mindestens einem der Ansprüche 1 und 2, dadurch gekennzeichnet, daß in den allgemeinen Formeln (1) und (2) Ar den Rest des Benzols darstellt.

4. Verfahren nach mindestens einem der Ansprüche 1 bis 3, dadurch gekennzeichnet, daß man mit wäßrigen Lösungen von Katalysatoren der in Anspruch 1 genannten allgemeinen Formel (3), in welcher M Zink bedeutet, kontinuierlich hydrolysiert.

5. Verfahren nach mindestens einem der Ansprüche 1 bis 4, dadurch gekennzeichnet, daß man mit wäßrigen Lösungen von Zinkchlorid als Katalysator kontinuierlich hydrolysiert.

6. Verfahren nach mindestens einem der Ansprüche 1 bis 5, dadurch gekennzeichnet, daß man bei Temperaturenvon 100° bis 140°C kontinuierlich hydrolysiert.

7. Verfahren nach mindestens einem der Ansprüche 1 bis 6, dadurch gekennzeichnet, daß die Konzentration der wäßrigen Lösung des Katalysators 1,2 bis 15 % beträgt.

8. Verfahren nach mindestens einem der Ansprüche 1 bis 7, dadurch gekennzeichnet, daß das zur Hydrolyse erforderliche Wasser in stöchiometrischer Menge oder in einem Überschuß bis zu etwa 100 %, bezogen auf die Äquivalente zu hydrolisierendem Chlor in der Ausgangsverbindung der in Anspruch 1 genannten Formel (2), eingesetzt wird.

9. Verfahren nach mindestens einem der Ansprüche 1 bis 8, dadurch gekennzeichnet, daß das zur Hydrolyse erforderliche Wasser in einem Überschuß bis zu 15 % über die stöchiometrische Menge hinaus, bezogen auf die Äquivalente zu hydrolysierendem Chlor in der Ausgangsverbindung der in Anspruch 1 genannten allgemeinen Formel (2), eingesetzt wird.

10. Verfahren nach mindestens einem der Ansprüche 1 bis 9, dadurch gekennzeichnet, daß man die kontinuierliche Hydrolyse in einer Rührkesselkaskade, bestehend aus 2 bis 5 hintereinander geschalteten, heizbaren Rührkessein, durchführt.

11. Verfahren nach mindestens einem der Ansprüche 1 bis 10, dadurch gekennzeichnet, daß man die kontinuierliche Hydrolyse in einer Rührkesselkaskade, bestehend aus 2 bis 3 hintereinander geschalteten, heizbaren Rührkesseln, durchführt.

12. Verfahren nach mindestens einem der Ansprüche 10 und 11, dadurch gekennzeichnet, daß man in den ersten Kessel der Kaskade den dichlormethylsubstituierten Aromat und wäßrige Katalysatorlösung kontinuierlich zudosiert und nach Bedarf in die weiteren Kessel zusätzlich Wasser oder wäßrige Katalysatorlösung eindosiert.

13. Verfahren nach mindestens einem der Ansprüche 10 bis 12, dadurch gekennzeichnet, daß die Verweilzeit in den einzelnen Kesseln etwa 30 bis etwa 240 min. beträgt, wobei die Verweilzeit in den einzelnen Kesseln unterschiedlich sein kann.

14. Verfahren nach mindestens einem der Ansprüche 10 bis 13, dadurch gekennzeichnet, daß die Verweilzeit in den einzelnen Kesseln etwa 40 bis etwa 80 min. beträgt.

15. Verfahren nach mindestens einem der Ansprüche 1 bis 14, dadurch gekennzeichnet, daß man anstelle der reinen dichlormethylsubstituierten Aromaten der in Anspruch 1 genannten allgemeinen Formel (2) eine Mischung dieser Aromaten mit bis zu etwa 50 Gew.% der entsprechenden trichlormethylsubstituierten Aromaten einsetzt.

16. Verfahren nach mindestens einem der Ansprüche 1 bis 15, dadurch gekennzeichnet, daß man anstelle der reinen dichlormethylsubstituierten Aromaten der in Anspruch 1 genannten allgemeinen Formel (2) eine Mischung dieser Aromaten mit etwa 0,5 bis etwa 30 Gew.% der entsprechenden trichlormethylsubstituierten Aromaten einsetzt.

17. Verfahren nach mindestens einem der Ansprüche 1 bis 16, dadurch gekennzeichnet, daß man vor oder nach einer destillativen Reinigung des aromatischen Aldehyds die aromatische Carbonsäure aus dem Reaktionsgemisch oder dem Destillationssumpf durch kontinuierliche Alkali-Wäsche abtrennt und durch Ansäuern in reiner Form ausfällt.

## Claims

1. A process for the continuous production of aromatic aldehydes of the formula (1) 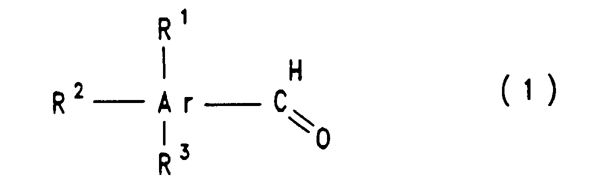 in which Ar is an aryl radical having from 6 to 14 carbon atoms, R¹, R² and R³ independently of one another are hydrogen, fluorine, chlorine or bromine atoms, which comprises continuously hydrolyzing dichloromethyl-substituted aromatics of the formula (2) 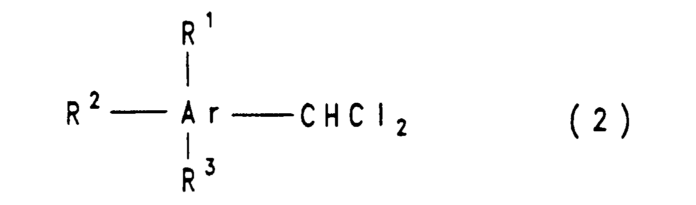 in which Ar, R¹, R², R³ have the above meanings, by metering in all of the catalyst in the form of an aqueous solution of one or more catalysts of the formula (3)
MXₙ (3)
in which M is a transition metal selected from the group consisting of nickel, copper and zinc, X is F, Cl, Br, I, OH, SO₄, PO₄ or NO₃, and n depending on the oxidation state of the transition metal M is the number 1, 2, 3 or 4, in a concentration of from 1 to 50%, based on the weight of water in the catalyst solution, at temperatures of from 70° to 160°C.

2. The process as claimed in claim 1, wherein Ar in the formulae (1) and (2) is the radical of benzene, naphthalene, anthracene or phenanthrene.

3. The process as claimed in at least one of claims 1 and 2, wherein Ar in the formulae (1) and (2) is the radical of benzene.

4. The process as claimed in at least one of claims 1 to 3, wherein hydrolysis is performed continuously with aqueous solutions of catalysts of the formula (3) given in claim 1, in which M is zinc.

5. The process as claimed in at least one of claims 1 to 4, wherein hydrolysis is performed continuously with aqueous solutions of zinc chloride as catalyst.

6. The process as claimed in at least one of claims 1 to 5, wherein hydrolysis is performed continuously at temperatures of from 100° to 140°C.

7. The process as claimed in at least one of claims 1 to 6, wherein the concentration of the aqueous solution of the catalyst is from 1.2 to 15%.

8. The process as claimed in at least one of claims 1 to 7, wherein the water required for hydrolysis is used in the stoichiometric amount or in an excess of up to about 100%, based on the equivalents of chlorine to be hydrolyzed in the starting compound of the formula (2) given in claim 1.

9. The process as claimed in at least one of claims 1 to 8, wherein the water required for hydrolysis is used in an excess of up to 15% above the stoichiometric amount, based on the equivalents of chlorine to be hydrolyzed in the starting compound of the formula (2) given in claim 1.

10. The process as claimed in at least one of claims 1 to 9, wherein the continuous hydrolysis is carried out in a cascade of stirred reactors comprising from 2 to 5 heatable stirred reactors connected in series.

11. The process as claimed in at least one of claims 1 to 10, wherein the continuous hydrolysis is carried out in a cascade of stirred reactors comprising from 2 to 3 heatable stirred reactors connected in series.

12. The process as claimed in at least one of claims 10 and 11, wherein the dichloromethyl-substituted aromatic and the aqueous catalyst solution are continuously metered into the first reactor of the cascade and water or aqueous catalyst solution is additionally metered into the further reactors as needed.

13. The process as claimed in at least one of claims 10 to 12, wherein the residence time in the individual reactors is from about 30 to about 240 minutes, the residence time in the individual reactors being able to be different.

14. The process as claimed in at least one of claims 10 to 13, wherein the residence time in the individual reactors is from about 40 to about 80 minutes.

15. The process as claimed in at least one of claims 1 to 14, wherein in place of the pure dichloromethyl-substituted aromatics of the formula (2) given in claim 1 a mixture of these aromatics with up to about 50% by weight of the corresponding trichloromethyl-substituted aromatics is used.

16. The process as claimed in at least one of claims 1 to 15, wherein in place of the pure dichloromethyl-substituted aromatics of the formula (2) given in claim 1 a mixture of these aromatics with from about 0.5 to about 30% by weight of the corresponding trichloromethyl-substituted aromatics is used.

17. The process as claimed in at least one of claims 1 to 16, wherein before or after a distillative purification of the aromatic aldehyde the aromatic carboxylic acid is separated off from the reaction mixture or the distillation residue by continuous alkali scrubbing and is precipitated in pure form by acidification.

## Revendications

1. Procédé pour la préparation en continu d'aldéhydes aromatiques de formule générale (1) 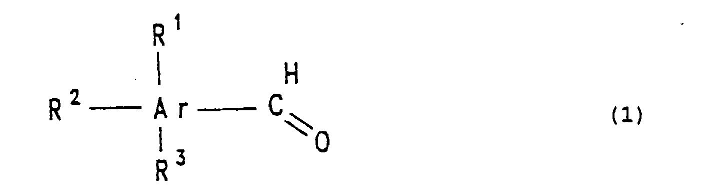 dans laquelle Ar représente un radical aryle avec 6 à 14 atomes de carbone, R¹, R² et R³, indépendamment les unp des autres, représentent des atomes d'hydrogène, de fluor, de chlore ou de brome, caractérisé en ce que l'on hydrolyse en continu des composés aromatiques substitués par dichlorométhyle de formule générale (2) 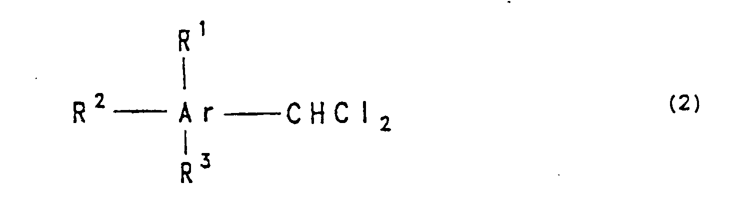 dans laquelle Ar, R¹, R², R³ ont les significations données ci-dessus, par addition progressive de tout le catalyseur sous forme d'une solution aqueuse d'un ou plusieurs catalyseurs de formule générale (3)
MXₙ (3)
dans laquelle M représente un métal de transition, de la famille du nickel, du cuivre et du zinc, X représente F, Cl, Br, I, OH, SO₄, PO₄ ou NO₃, et n, en fonction du degré d'oxydation du métal de transition M, vaut 1, 2, 3 ou 4, d'une concentration de 1 à 50 % par rapport au poids de l'eau dans la solution du catalyseur, à des températures de 70° à 160°C.

2. Procédé selon la revendication 1, caractérisé en ce que dans les formules générales (1) et (2) Ar représente le radical du benzène, du naphtalène, de l'anthracène ou du phénanthrène.

3. Procédé selon au moins l'une des revendications 1 et 2, caractérisé en ce que dans les formules générales (1) et (2), Ar représente le radical du benzène.

4. Procédé selon au moins l'une des revendications 1 à 3, caractérisé en ce que l'on met en oeuvre l'hydrolyse en continu avec des solutions aqueuses de catalyseurs de formule générale (3) citée dans la revendication 1, dans laquelle M représente le zinc.

5. Procédé selon au moins l'une des revendications 1 à 4, caractérisé en ce que l'on met en oeuvre l'hydrolyse en continu avec des solutions aqueuses de chlorure de zinc comme catalyseur.

6. Procédé selon au moins l'une des revendications 1 à 5, caractérisé en ce que l'on met en oeuvre l'hydrolyse en continu à des températures de 100° à 140°C.

7. Procédé selon au moins l'une des revendications 1 à 6, caractérisé en ce que la concentration de la solution aqueuse du catalyseur est de 1,2 à 15 %.

8. Procédé selon au moins l'une des revendications 1 à 7, caractérisé en ce que l'on utilise l'eau nécessaire à l'hydrolyse en quantité stoechiométrique ou en un excès allant jusqu'à environ 100 %, par rapport à l'équivalent de chlore à hydrolyser dans le composé de départ de formule (2) citée dans la revendication 1.

9. Procédé selon au moins l'une des revendications 1 à 8, caractérisé en ce que l'on utilise l'eau nécessaire à l'hydrolyse en un excès allant jusqu'à 15 % au-dessus de la quantité stoechiométrique, par rapport à l'équivalent de chlore à hydrolyser dans le composé de départ de formule générale (2) citée dans la revendication 1.

10. Procédé selon au moins l'une des revendications 1 à 9, caractérisé en ce que l'on met en oeuvre l'hydrolyse en continu dans une cascade de cuves à agitation, constituée de deux à trois cuves à agitation chauffables, installées l'une après l'autre.

11. Procédé selon au moins l'une des revendications 1 à 10, caractérisé en ce que l'on met en oeuvre l'hydrolyse en continu dans une cascade de cuves à agitation, constituée de deux à trois cuves à agitation chauffables, installées l'une après l'autre.

12. Procédé selon au moins l'une des revendications 10 et 11, caractérisé en ce que l'on ajoute progressivement en continu à la première cuve de la cascade le composé aromatique substitué par dichlorométhyle et la solution aqueuse de catalyseur et, selon les besoins, on ajoute progressivement à la cuve suivante en outre de l'eau ou de la solution aqueuse de catalyseur.

13. Procédé selon au moins l'une des revendications 10 à 12, caractérisé en ce que la durée de séjour dans les différentes cuves est d'environ 30 à environ 240 minutes, la durée de séjour dans les différentes cuves pouvant être différente.

14. Procédé selon au moins l'une des revendications 10 à 13, caractérisé en ce que la durée de séjour dans les différentes cuves est d'environ 40 à environ 80 minutes.

15. Procédé selon au moins l'une des revendications 1 à 14, caractérisé en ce que, au lieu des composés aromatiques purs substitués par dichlorométhyle de formule générale (2) citée dans la revendication 1, on utilise un mélange de ces composés aromatiques avec une teneur en composés aromatiques correspondants substitués par trichlorométhyle allant jusqu'à environ 50 % en poids.

16. Procédé selon au moins l'une des revendications 1 à 15, caractérisé en ce que, au lieu des composés aromatiques purs substitués par dichlorométhyle de formule générale (2) citée dans la revendication 1, on utilise un mélange de ces composés aromatiques ayant une teneur d'environ 0,5 à environ 30 % en poids des composés aromatiques correspondants substitués par trichlorométhyle.

17. Procédé selon au moins l'une des revendications 1 à 16, caractérisé en ce que, avant ou après une purification par distillation de l'aldéhyde aromatique, on sépare l'acide carboxylique aromatique du mélange réactionnel ou du résidu de distillation par lavage alcalin en continu et on le précipite par acidification sous forme pure.
